# EUROPEAN PATENT APPLICATION

(11) **EP 1 747 798 A2**
(43) Date of publication of application: **31.01.2007**
(21) Application number: 06380218.5
(22) Date of filing: 28.07.2006
(51) Int. Cl.: A61N 2/02

(54) **Electromagnetical topical system**

(30) Priority: 28.07.2005 ES 200501853
(71) Applicant: Poyatos Pérez, Manuel, 14500 Puente Genil (ES)
(72) Inventor: Pinel Jiménez, Marcos, 41007 Sevilla (ES)
(74) Representative: Carvajal y Urquijo, Isabel

(57) **Abstract**

The invention relates to an electromagnetic topical system for treating skin diseases, formed by a treatment unit (1) where a user is introduced, and a series of fluid emission (6) and reception (7) elements, the molecules of which fluid are oriented and charged electromagnetically by means of a coil (2) generating pulsating fields and a series of anodes and cathodes.

A system of pumps (5, 10, 15, 20), valves (11, 21) and pipes of non-magnetic material supply the fluid from one or more deposits (3, 13) where it is stored and treated.

The system further comprises a series of filters (9, 19) and a series of bleed valves (12) for removing skin flakes or particles suspended in the fluid.

## Description

### Field of the Invention

This invention relates to a system of treating skin conditions, such as psoriasis for example, by means of electromagnetic properties of particles immersed in a fluid.

More specifically, the treatment relates to a bath in a fluid, preferably water, to which electromagnetic fields are applied to use the healing properties of these fields, known as magnet therapy.

### State of the Art

The field of the art in which the invention is comprised is that of the treatment of skin diseases by means of baths with healing properties using electromagnetic properties.

There are several documents disclosing probes or magnet therapy apparatus. Document ES 2129215 T3 can thus be mentioned, relating to a flexible electrode for treating the skin by electromagnetic fields, or document ES 2005926 in which the apparatus stimulating the skin is a rigid structure also affecting a reduced area.

These two inventions have as a main drawback the fact that the treatment surface is particularly small, so the time it takes to treat any somewhat generalized disease is particularly long.

### Description of the Invention

This invention relates to an electromagnetic topical system which simultaneously treats all the skin of the user so the speed of the treatment is particularly high.

The invention relates to an electromagnetic topical system for treating skin diseases, such as psoriasis for example, by immersing the user in a fluid charged with and within an electromagnetic field.

To that end the user is introduced in a treatment unit formed by a rectangular prism or cylinder, or any other shape, having a size that is sufficient so that the user may comfortably remain upright or lying down. Fluid emission elements and a series of reception elements are arranged in the treatment unit, allowing the circulation of said fluid.

These emission and reception elements perform a second function as they have a first and second screen acting as anodes and cathodes of an electric field between 25 and 45 mV.

The number of emission and reception elements is variable, the installation of several planar elements being recommended, grouping each type of element on one side of the inner wall of the treatment unit.

The bottom of the treatment unit is perforated for the complete disposal of the fluid, there being a small chamber where the fluid disposal devices and valves are connected.

A coil generating pulsating magnetic fields surrounding the treatment unit moves in a longitudinal direction of the treatment unit to vary the position from where the electromagnetic field is emitted with intermittent stops.

This coil generating pulsating magnetic fields can move throughout a series of racks of a non-magnetic material located on the outer wall of the treatment unit, for example four racks located in positions equally spaced from one another, on which the gears joined to the coil by its shaft are supported.

According to one embodiment, at least one storage deposit is arranged where the charging and orientation of the fluid molecules is carried out, generally in periods of between one and three days, by means of an orientation coil and a second electric field generated by one or more anodes and one or more cathodes. This electric field is variable, although it is recommended that it have values between 50 and 90 mV.

The circuit which allows carrying the fluid from the deposit to the treatment unit and making it circulate between the emission and reception elements consists of:
o A three-way valve for selecting the type of fluid movement, whether from the deposit to the treatment unit or between the emission and reception elements.
ο A first pump providing the hydraulic load necessary for both types of movements, this first pump being located between the three-way valve and the emission elements.
o A second pump carrying the fluid from the perforated bottom of the treatment unit to the deposit, providing the hydraulic load necessary for the fluid to furthermore traverse an assembly of filters that remove the skin flakes and any other particle present in the fluid.

All these elements are made of a non-magnetic material and are joined to one another by means of non-magnetic pipes, such as PVC pipes.

An assembly of bleed valves is located at points where skin flakes or particles may accumulate, such as at the perforated bottom of the treatment unit, in front of the filters, at the outlet of the reception elements and in the deposit.

More than one storage deposit can be arranged for each treatment unit, for which the circuit must be doubled, installing new pumps, bleed valves, filters,... arranging a parallel feed circuit.

In any case it is necessary to arrange circuit control valves, for example to control the deposit that is activated, or to close off fluid access to each element of the circuit and to thus be able to easily carry out its maintenance.

Many types of fluid can be used, the use of purified demineralized water being recommended, and the water must be replaced as it is lost through the bleed valves or in any other way.

It is also recommended to periodically change the fluid.

### Description of the Drawings

The following figures are included to better understand the invention:
Figure 1 shows the flow diagram of an embodiment of the invention.
Figure 2 shows an embodiment of the treatment unit.

### Description of an Embodiment

An embodiment of the invention is very briefly described below as an illustrative and non-limiting embodiment thereof.

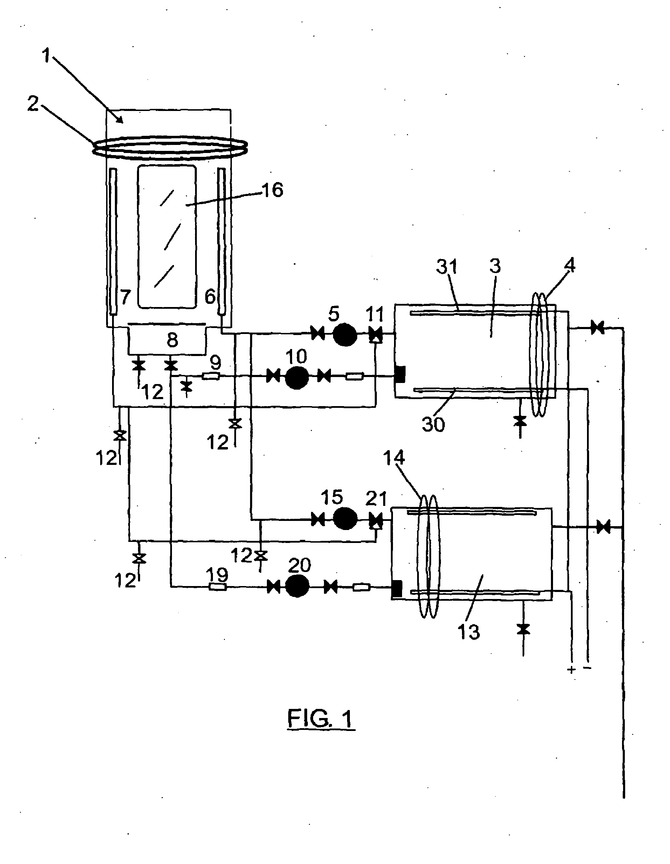

Figure 1 shows the flow diagram of the electromagnetic topical system. A storage deposit (3) surrounded by orientation coils (4) generating an electromagnetic field inside the storage deposit (3) can be seen in said Figure. The molecules of a fluid contained in the deposit (3) are charged and oriented with these electromagnetic fields. The fluid will generally be water which has undergone purification and demineralization processes although other fluids can also be used.

Anodes (31) and cathodes (30) are introduced in the deposit (3) to complement the charging and molecular orientation generated by the orientation coils (4), arranging between them an electric field, for example between 50 and 90 mV, the latter charge corresponding to that present in a healthy epidermal cell.

The charged fluid is carried by means of conduits made of PVC pipes or of another non-magnetic material, through a three-way valve (11) and a first non-magnetic pump (5) to emission elements (6), one of which is shown in Figure 1, located inside a treatment unit (1). One or more emission elements (6) will generally be installed having a considerably planar shape and aligned on one end of the treatment unit (1), each one preferably having a first metallic screen (36) at its emission opening. One or more reception elements (7), also aligned, are located at the opposite end, being able to arrange a second metallic screen (37) at the inlet.

It is recommended that both the emission elements (6) and the reception elements (7) maintain a considerably vertical structure.

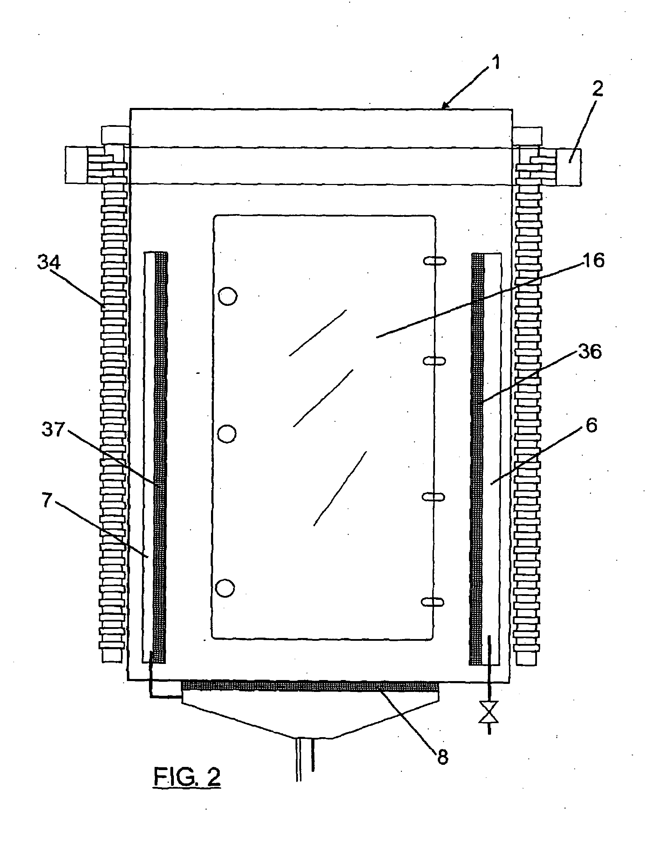

An example of a treatment unit (1) is shown in Figure 2. The user of the electromagnetic topical system receives his/her treatment in this treatment unit (1), being immersed in an electrically charged fluid bath which transmits its energy to the unbalanced cell system. For this reason it has dimensions such that any user may comfortably remain upright if the treatment unit (1) is vertical, or lying down if the arrangement of the treatment unit (1) is horizontal. Furthermore it is advisable that the user is not in direct contact with the emission elements (6) or reception elements (7).

For example, the treatment unit (1) can be carried out in a non-magnetic cylinder or tube of methacrylate or the like, with about 1 m in diameter and 2 meters in length. A door (16) for the user to enter will be made therein, assuring the leak-tight sealing of the door (16) at least in the parts that are immersed.

Another possibility is to make the treatment unit (1) with the shape of a 2.5 m x 0.7 m x 0.5 m rectangular prism or with similar dimensions. This rectangular prism can be folded such that the user is comfortably lying down while his/her treatment is carried out. To enter inside, the upper wall of the prism can be opened or a door (16) can be made on one side.

Once the user has entered and suitably closed the door (16), pumping of the charged fluid begins with the first pump (5) and the fluid is introduced in the treatment unit (1) through the emission elements (6) up to a level defined by the user's height and treatment needs.

During the user's treatment, the fluid is taken up by the reception elements (7), which carry the fluid to the three-way valve (11), and is re-introduced into the treatment unit (1) by the emission elements (6)

In order to recharge the energy transmitted from the fluid to the user's cells, the treatment unit (1) is surrounded by a coil (2) generating pulsating electromagnetic fields which performs operating cycles with stops between 30 and 120 seconds, depending on the patient's need and severity.

This coil (2) moves along a longitudinal axis of the treatment unit (1). The coil (2) begins emitting pulsating electromagnetic fields in a first position, moving from such position after each operating cycle, so as to vary the incidence of the magnetic fields on the user.

The movement of the coil can be carried out, for example, by means of non-metallic guide racks (34) arranged on the outer wall of the treatment unit (1). For example, four racks (34) can be arranged in positions equally spaced from one another in the case of a vertical cylindrical treatment unit (1), or in the four vertical edges in the case of a rectangular prismatic treatment unit (1).

The first screen (36) of the emission elements (6) may further be made to act as an anode of an electric field between 25 and 45 mV, whereas the second screen (37) acts as a cathode (or vice versa). The electric field distribution is thus improved, in turn improving treatment efficacy.

Perforations are made at the bottom (8) of the treatment unit (1) so as to allow the emptying of the treatment unit (1). Once the fluid traverses the bottom, it enters a non-metallic pipe and passes through an assembly of filters (9) to remove skin flakes and any other material present in the fluid. These filters (9) can be, for example, a series of mesh filters with a last screen pore diameter of half a micron.

A second pump (10) is arranged behind the filters (9) that reintroduces the fluid into the storage deposit (3) where it remains between 24 and 48 hours to allow introducing the initially mentioned charge in the molecules.

Bleed valves (12) can be arranged at several points of the circuit, for example under the perforated bottom (8) of the treatment unit (1), before the filters (9), in the deposit (3), and at the outlet of the emission elements (7), to remove the skin flakes and other particles accumulating at said points of the circuit.

However, it is necessary to provide a fresh water supply, for example directly to the treatment deposits (3, 13).

A second circuit can be installed in shunt with a second deposit (13), second orientation coils (14), and the pumps (15, 20), pipes and filters (19) necessary to supply the treated fluid to the treatment unit (1), making it circulate between the emission (6) and reception (7) elements through a three-way valve (21), taking it up again at the end of the treatment, filtering it and reinitiating the process.

Even more circuits can be added in shunt to this basic installation, although it is recommended to limit them to two so as not to excessively increase the cost of the system.

An assembly of valves also made of a non-magnetic material regulates the fluid circulation, allowing fluid circulation through a single circuit or through both at the same time.

A doctor in charge of the treatment shall establish, with the aid of diagnostic methods such as an oscilloscope, the user's treatment needs to use the electromagnetic topical system, establishing:
- The time the user is inside the treatment unit (1), which will generally range between 10 and 15 minutes.
- Down time of the coil (2) generating pulsating electromagnetic fields.
- Power of said coil (2).
- Electric field created between the anodes and cathodes inside the treatment unit (1).
- Level of the fluid inside the treatment unit (1).

## Claims

1. An electromagnetic topical system for treating skin diseases, of the type consisting of subjecting the patient's epidermis to an electromagnetic field generated by a coil and an anode and cathode assembly, **characterized in that** it comprises:
a hollow treatment unit (1) where a user of the system is introduced, in turn comprising:
at least one fluid emission element (6) and one reception element (7) located in inner walls of the treatment unit (1); and
a coil (2) generating pulsating magnetic fields, movable along a longitudinal direction of the treatment unit (1).

2. A topical system according to claim 1, **characterized in that** it further comprises:
at least one deposit (3, 13) where the charging and electromagnetic orientation of the fluid molecules is carried out by means of an orientation coil (4, 14), an anode (31) and a cathode (30) between which there is arranged an electric field between 50 and 90 mV;
at least one three-way valve (11, 21) for regulating the fluid circulation between the deposit (3, 13) and the emission elements (6) or between the emission elements (6) and the reception elements (7);
at least a first pump (5, 15) for making the fluid going through the three-way valve (11, 21) circulate;
at least a second pump (10) for reintroducing the fluid of the treatment unit (1) in the deposit (3, 13); and
at least one assembly of filters (9, 19) located between a perforated bottom (8) of the treatment unit (1) and the second pump (10) for removing the skin flakes and other particles present in the fluid.

3. A topical system according to claim 2, **characterized in that** the emission elements (6) and the reception elements (7) comprise a first and second screen (36, 37) acting as anode and cathode of the treatment unit (1).

4. A topical system according to any of claims 1 to 3, **characterized in that** arranged between the anode and cathode of the treatment unit (1) there is a field of 25 to 45 mV.

5. A topical system according to any of claims 1 to 4, **characterized in that** it comprises a plurality of emission elements (6) according to an arrangement along an inner wall of the treatment unit (1) and a plurality of reception elements (7) in an arrangement along the opposite inner wall of the treatment unit (1).

6. A topical system according to any of claims 1 to 5, **characterized in that** the treatment unit (1) comprises a cylinder of non-magnetic material of about 1 meter in diameter and 2 meters in length, where a leak-tight door is carried out so that the user can enter inside.

7. A topical system according to any of claims 1 to 5, **characterized in that** the treatment unit (1) comprises a rectangular prism of non-magnetic material of dimensions of about 2.5 m long, 0.7 m wide and 0.5 m high, where a leak-tight door is carried out so that the user can enter inside in one of its larger sides.

8. A topical system according to any of claims 6 or 7, **characterized in that** the treatment unit (1) is arranged in a considerably vertical manner.

9. A topical system according to any of claims 6 or 7, **characterized in that** the treatment unit (1) is arranged in a considerably horizontal manner.

10. A topical system according to any of claims 1 to 9, **characterized in that** the fluid is purified and demineralized water.

11. A topical system according to any of claims 2 to 10, **characterized in that** bleed valves (12) are located in front of the filters (9, 19), on the perforated bottom (8) of the treatment unit (1), in the deposits (3, 13), and at the outlet of the reception elements (7).
